(19) **Europäisches Patentamt**
**European Patent Office**
**Office européen des brevets**

(11) **EP 3 375 434 A1**

(12) **EUROPEAN PATENT APPLICATION**

(43) Date of publication:
**19.09.2018 Bulletin 2018/38**

(51) Int Cl.:
**A61K 8/99** (2017.01)  **A61K 8/73** (2006.01)
**A61Q 19/00** (2006.01)  **A61P 17/02** (2006.01)
**A61P 17/06** (2006.01)  **A61K 35/74** (2015.01)

(21) Application number: **18169832.5**

(22) Date of filing: **09.10.2009**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **13.10.2008 ES 200802885**

(62) Document number(s) of the earlier application(s) in accordance with Art. 76 EPC:
**09737349.2 / 2 337 556**

(71) Applicant: **Lipotec, S.A.**
**08850 Gavà-Barcelona (ES)**

(72) Inventors:
• **GARCÍA ANTÓN, Jose María**
**08017 Barcelona (ES)**

• **ONCINS BERGAS, Carla**
**08028 Barcelona (ES)**
• **PÉREZ ARCAS, Román**
**08940 Cornellá de Llobregat (ES)**

(74) Representative: **Carvajal y Urquijo, Isabel et al Clarke, Modet & Co.**
**Suero de Quiñones, 34-36**
**28002 Madrid (ES)**

Remarks:
This application was filed on 27.04.2018 as a divisional application to the application mentioned under INID code 62.

(54) **COSMETIC OR DERMOPHARMACEUTICAL COMPOSITION CONTAINING PSEUDOALTEROMONAS FERMENT EXTRACT**

(57) Cosmetic or dermopharmaceutical compositions for the treatment and/or care of the skin, scalp and/or nails, preferably to improve the barrier function of the skin, scalp and/or nails, and specifically for the treatment and/or care of those conditions, disorders and/or pathologies of the skin, scalp and/or nails associated with an an alteration in the keratinization process.

EP 3 375 434 A1

Description

## FIELD OF THE INVENTION

The present invention relates to the use of *Pseudoalteromonas* ferment extract [INCI: Pseudoalteromonas Ferment Extract] in the preparation of cosmetic or dermopharmaceutical compositions for the treatment and/or care of the skin, scalp and/or nails, preferably for the treatment and/or care of those conditions, disorders and/or pathologies of the skin, scalp and/or nails associated with an altered keratinization process.

## BACKGROUND OF THE INVENTION

The epidermis constitutes the outermost region of the skin tissue. Its thickness usually does not exceed 0.2 mm, but it encompasses an ordered mass of stratified cells that should be differentiated into two areas: the more superficial one called the stratum corneum, which is formed by superimposed layers of inert and hard cells called corneocytes. Corneocytes usually undergo controlled desquamation; and the deeper one formed by live cells, called keratinocytes. Part of the keratinocytes undergo a controlled proliferation process, while the remaining ones form morphologically different cell strata in the process of differentiation into basal, prickle, granular and lucid cells, the latter being only on the skin of the palms and soles. This global process ends with the conversion of keratinocytes into corneocytes ["Ciencia Cosmética: Bases fisiológicas y criterios prácticos" (Cosmetic Science: Physiological Bases and Practical Criteria), L. Pons, J.L. Parra, Consejo General de Colegios Farmacéuticos, 36-37 (1995)].

The epidermis also keratinizes to produce and regenerate nails and hair. Keratinization involves skin cell maturation and migration, being started at the innermost layer of the epidermis. Keratinocytes accumulate, travel and become differentiated throughout the following layers of the epidermis. In the stratum corneum, the keratinocytes complete their keratinization process, forming anucleated, polyhedral and flat corneocytes.

The corneocytes are separated by lipid layers. Each corneocyte is made up of a group of keratin filaments inside an insoluble protein sheath. In the outer part of this sheath, long-chain ceramides are found chemically bound to the proteins that interact with the intercellular lipid layers, which together result in a hydrophobic structure. Additionally, natural moisturizing factor (NMF) molecules are found both intracellularly and extracellularly in the stratum corneum, which retain water due to of their hygroscopic nature.

The lipid composition of the stratum corneum varies among different anatomical regions of the skin and is also dependent on the age and the season. Ceramides, cholesterol and fatty acids are proportionally lower in the winter and spring than in the summer. This lipid ratio affects skin barrier function, water retention and desquamation ["The Biology of the Skin", R.K. Freinkel and D.T. Woodley eds., Parthenon Publishing, 221 (2001)].

This structure mainly functions as an outer skin barrier, and therefore this corneocyte and lipid structure regulates transepidermal water loss (TEWL). Transepidermal water loss through the stratum corneum of the skin is inherent to normal stratum corneum function, because the outermost layers of the stratum corneum must be hydrated to maintain flexibility, and it is through these layers how water loss occurs. Having to hydrate the outermost layers of the stratum corneum causes a water gradient within the stratum corneum, where the percentage of water ranges from less than 20% in the outermost layers to over 40% in the innermost layers of the stratum corneum ["Bioengineering of the Skin: Water and the Stratum Corneum", P. Eisner, E. Berardesca and J.H. Maibach, eds., CRC Press, 3-12 (1994)]. When transepidermal water loss increases, the skin becomes dehydrated which is reflected in an approximate 10% decrease in the percentage of water in the layers of the stratum corneum, with less flexibility and frequent desquamation.

The existence of an effective cohesion between epidermal cells, both at a keratinocyte and corneocyte level, is of great importance for maintaining skin physiology within normal limits. Normal desquamation of the most superficial corneocytes is a vital need not only for maintaining the aesthetic appearance of the epithelial tissue. Moreover it solves a good part of the problems that are frequently triggered when our body comes into contact with the hostile environment, but also for guaranteeing, in many cases, an adequate protection against most physical, chemical and biological aggressions that pose a health threat. This normal desquamation requires constant controlled cell renewal which begins in the basal stratum of the epidermis. Cells have to circulate through epidermal strata to reach the stratum corneum when they have undergone proper differentiation. The mitotic rhythm of proliferating keratinocytes allows for new keratinocytes to be continually incorporated into the epidermis, theoretically in a sufficient number to replace the number of corneocytes eliminated ["Ciencia Cosmética: Bases fisiológicas y criterios prácticos", L. Pons, J.L. Parra, Consejo General de Colegios Farmacéuticos, 71 (1995)].

Disorders of the epidermis influence its barrier function and may alter the lipid or protein composition of the stratum corneum or lead to a defective stratum corneum by altering keratinocyte proliferation ["The Biology of the Skin", R.K. Freinkel and D.T. Woodley eds., Parthenon Publishing, 225 (2001)]. There are numerous cutaneous problems that are characterized by defective epidermal differentiation, that is, dysfunction of epidermal cell proliferation and/or differentiation and/or migration, especially keratinocytes. For example, proliferation problems may cause the production of a thick stratum corneum and abnormal desquamation, i.e. hyperkeratosis, which may or may not indicate a pathology depending on its severity. Hyperkeratosis is the result of an increased production or decreased elimination of keratinized cells; the general term hyperkeratosis includes both orthokeratosis (increase of completely keratinized cells) and also parakeratosis (increase of partially keratinized cells). Alterations in the keratinization process may lead to, or be the result of, disorders and/or pathologies such as, for example, reactive hyperkeratosis, palmar and/or plantar hyperkeratosis, calluses or corns, actinic keratosis, non-actinic keratosis (keratosis pilaris, keratosis punctata, keratosis senilis, keratosis striata), atopical dermatitis, contact eczema, seborrheic dermatitis, dandruff, cradle cap in infants, acne, rosacea, nevus, ichthyosis, psoriasis, parakeratosis, pityriasis, lichen planus, palmoplantar keratoderma (*keratosis palmaris et plantaris*), Darier's disease, onychomycosis, dermatomycosis, and certain benign tumor proliferations, such as papillomas or warts, keratoacanthoma or seborrheic keratosis, or malignant tumors. In addition to the aforementioned disorders and/or pathologies, there are skin and/or nail conditions associated with keratinocyte proliferation and/or differentiation, for example, dry skin or xerosis which is very common among the general population, or conditions given in special populations such as newborns or the elderly. Elderly people for example suffer from less cohesive corneocytes, less water retained by the stratum corneum and furthermore ungual hyperkeratosis. Xerosis results in roughness, desquamation, loss of flexibility, hyperkeratosis, fissures, inflammation and pruritus.

Although hyperkeratosis may occur on any part of the skin, there are certain areas that are most often affected such as elbows, knees, palm hands and sole feet, and especially the heels. It should be mentioned that pathologies and/or disorders symptoms of the skin feet include hardening of the sole feet skin with the formation of fissures, pain and even bleeding. Palmoplantar keratoderma is a condition that affects the hands and feet and that can be genetic in origin or acquired by changes in a person's health or environment, such as climate keratoderma in postmenopausal women.

Cracked heels, or plantar hyperkeratosis (or *keratoderma plantare sulcatum* or xeorosis) is a very common disorder in response to environmental aggressions such as cold or dryness and/or mechanical causes, such as for example, weight, obesity, pressure or friction, walking barefoot or with open shoes. Although it is mainly an aesthetic inconvenience, it is also a significant problem in certain pathologies involving insensitivity, poor blood circulation and foot injuries, such as diabetes ("diabetic foot") or vascular problems.

As consequence of the alteration of the skin barrier function produced in many of the previously mentioned conditions, disorders and/or pathologies, such as response, it is developed an altered permeability and high TEWL levels, xerosis and keratinocyte hyperproliferation.

Regulating keratinocyte proliferation and differentiation is necessary for the treatment of conditions, disorders and/or pathologies resulting from altered keratinization. Treatment with activator ligands of retinoid X receptor (RXR) like vitamin D and retinoic acid regulates keratinocyte differentiation. Activators of peroxisome proliferator-activated receptors (PPARs) also look to modulate keratinocyte differentiation in pathological epidermis, characterized by aberrant cell differentiation and hyperproliferation [L.G. Kömüves et a/., J. Invest. Dermatol., 115, 361-67 (2000)]. The beneficial effect of metalloproteinase inhibitor TIMP-2 on chronic dermatitis is also known [H. Miyoshi et al., J. Dermatol., 32, 346-53 (2005)]. TIMP-2 accelerates healing by increasing proliferation and migration of keratinocyte and dermal fibroblast, and it can also induce epidermal keratinocyte differentiation. It is also known in the state of the art that proteoglycans, components of the extracellular matrix, play an important role in the modulation of the structure and regulation of skin functions, influencing cell development and differentiation and wound healing [P.D. Gupta et al., Current Science, 78, 1-5 (2000)].

Conventional treatments for xerosis involve the topical application of moisturizing agents: emollients, occlusives and humectants. They help restore the barrier function of the epidermis, by covering small fissures, providing a calming protective film and increasing water content of the epidermis [J.N. Kraft et al., Skin Therapy Letter, 10, 1 (2005)].

Traditional methods to treat keratinization disorders and/or pathologies include moisturizing agents, emollients, desquamating agents or exfoliants and keratolytics to facilitate the removal of thick skin. The beneficial effect of urea and derivatives thereof, as well as glycerin, on hydration and skin barrier function recovery is known in the state of the art [J.W. Fluhr et al., Acta Derm. Venerol., 79, 418-21 (1999); M. Gloor, Skin Pharmacol. Physiol., 17, 267-73 (2004); K.C. Madison. J. Invest. Dermatol. 121, 231-41 (2003); G. Swanbeck, Acta Derm. Venerol., 48, 123-7 (1967)]. In the treatment of hyperkeratosis, in addition to urea, other desquamating agents such as salicylic acid or alpha-hydroxy acids like lactic or glycolic acids, have traditionally be used.

Several examples and combinations of desquamating agents and keratolytic agents can be found in literature which attempt to improve upon known treatments for hyperkeratotic skin, such as combinations of urea and propylene glycol (FR 2342059 A1, WO 87/04617 A1), the use of alpha- or beta- hydroxy acids or keto acids (US 3920835 A, US 3988470 A, US 4234599 A, US 4363815 A, US 5886042 A, US 6191167 A, EP 0232982 A1, EP 1032378 A1, US 5407958 A1, FR 2756489 A1, US 2006/0269495 A1, US 2006/026949 A1), or protease use (FR 2761362 A1, FR 2761363 A1, FR 2767833 A1, FR 2850024 A1, FR 2888494 A1, US 2003/0232042 A1, US 2003/0232043 A1).

In the state of the art, numerous active agents are known for the treatment of conditions, disorders and/or pathologies associated with an alteration of keratinization, such as, for example, cysteic acid derivatives (US 4224339 A), oxoacids (US 6071962 A, US 5847003 A), alpha-hydroxy and alpha ketoretinoic acids (US 4194007 A), *N*-acylethylenetriacetic acids (US 5728733 A, US 5621008 A), 8-hexadecen-1,16-dicarboxylic acid (FR 2888494 A1), L-carnitine esters with hydroxyacids (EP 0596838 A2, EP 0628308 A1, EP 0628309 A1, EP 0631779 A1), organometallic complexes (FR 2839449 A1), pyroglutamic acid esters (US 5045559 A), pH neutralizing agents of the stratum corneum (US 2003/0113352 A1); antioxidants like *N*-acetylcysteine and derivatives (US 2003/0229141 A1, WO 95/00136 A1), gallium pyrones (US 5747482 A), n-docosanol (US 5948822 A), sphingolipids with lysophosphatidic acid (US 2004/0138177 A1), oligosaccharide aldonic acids (US 2008/0090772 A1) or 2-oxothiazolidine-4-carboxylic acid (FR 2816838 A1), among many others.

However, despite the large number of active agents for the treatment of the conditions, disorders and/or pathologies associated with an alteration of keratinization, these active agents are not always completely effective, and complete remission of symptoms is sometimes slow and incomplete. In addition, in the case of regular therapeutic treatments of psoriasis or eczemas with topical corticosteroids, drug resistance is common. On the other hand, the use of acids and other keratolytics can be aggressive for the epidermis, resulting in irritation and photosensitivity. Therefore, there is a need to develop innocuous treatments that allow for a rapid remission of hyperkeratotic symptoms and/or that help prevent them from appearing.

Previously, we had shown the epithelizing properties of the glycoprotein produced by the *Pseudoalteromonas Antarctica* bacteria (ES 2181592 A1), which is marketed as Antarcticine® [INCI: Pseudoalteromonas Ferment Extract]. This glycoprotein also has other important properties for its cosmetic use: it promotes collagen and elastin synthesis, it improves skin moisturization and behaves as a cryoprotectant. This cryoprotective profile makes the molecule suitable for preventing dry skin under extremely cold conditions. This set of properties gives it, its known anti-wrinkle effect. *Pseudoalteromonas* ferment extract has also been demonstrated to selectively improve human dermal fibroblast adhesion and to promote cell growth of human epidermal keratinocytes. Its inclusion in fabrics to improve wounds and problems associated with "diabetic foot" has also been described (EP 1700947 A2).

However, although *Pseudoalteromonas* ferment extract's ability to stimulate keratinocyte proliferation is known, surprisingly we have found that it considerably improves the barrier function of the skin, scalp and/or nails, and particularly hyperkeratosis and symptoms associated with keratinization disorders and/or pathologies by means of the regulation of the balance between keratinocyte proliferation and differentiation.

## DESCRIPTION OF THE INVENTION

Therefore, in a first aspect, the present invention relates to a cosmetic or dermopharmaceutical composition that contains *Pseudoalteromonas* ferment extract for the treatment and/or care of conditions, disorders and/or pathologies associated with an alteration of the barrier function of the skin, scalp and/or nails, and preferably an alteration of the keratinization.

Preferably, conditions, disorders and/or pathologies of the skin, scalp and/or nails are associated with an alteration of the keratinization include dry skin, xerosis, hyperkeratosis, reactive hyperkeratosis, palmar and plantar hyperkeratosis, calluses or corns, actinic keratosis, non-actinic keratosis (keratosis pilaris, keratosis punctata, keratosis senilis, keratosis striata), atopical dermatitis and contact eczema, seborrheic dermatitis, dandruff, cradle cap in infants, acne, rosacea, nevus, ichthyosis, psoriasis, parakeratosis, pityriasis, lichen planus, palmoplantar keratoderma (*keratosis palmaris et plantaris*), Darier's disease, onychomycosis, dermatomycosis, certain benign tumor proliferations, such as papillomas or warts, keratoacanthoma or seborrheic keratosis, or malignant tumor proliferation, chronic wound formation (ulcers), hypertrophic wounds and keloids, or thinning and fragility of the epidermis (from abnormal healing, varicose veins or burns).

In a particular embodiment, *Pseudoalteromonas* ferment extract can be added to compositions by aqueous solution, or be solubilized in conventional cosmetically or dermopharmaceutically acceptable solvents such as, including but not limited to, ethanol, propanol, isopropanol, propylene glycol, glycerin, butylene glycol or polyethylene glycol or any combination thereof.

In another particular formulation, the cosmetically or dermopharmaceutically effective amount of *Pseudoalteromonas* ferment extract to be administered to treat a condition, disorder and/or pathology will depend on the severity of the disorder and/or pathology and frequency of administration. The frequency of application may vary widely depending on the needs of each subject, with recommendation for a range of application from once a month to ten times a day, preferably from once a week to four times a day, more preferably from three times a week to three times a day, even more preferably once or twice a day.

"Cosmetically or dermopharmaceutically effective amount" means an amount that is non-toxic but enough to provide the desired effect. *Pseudoalteromonas* ferment extract is used in the cosmetic or dermopharmaceutical composition of the present invention at cosmetically or dermopharmaceutically effective concentrations to obtain the desired effect; in a preferred way, considering the total weight of the composition, between 0.00000001% and 40% (in weight); preferably between 0.00001% and 30% (in weight), and more preferably between 0.0001% and 20% (in weight).

In another particular embodiment, the cosmetic or dermopharmaceutical composition of the present invention can also be incorporated to cosmetic or dermopharmaceutical delivery systems or sustained-release systems.

The term "delivery systems" relates to a diluent, adjuvant, excipient or carrier whereby the ferment extract from the composition of the invention is administered. These cosmetic or dermopharmaceutical carriers can be liquids, such as water, oils or surfactants, including those of a petroleum, animal, plant or synthetic origin, such as, including but not limited to, peanut oil, soybean oil, mineral oil, sesame oil, castor oils, polysorbates, sorbitan esters, sulfate esters, sulfates, betaines, glucosides, maltosides, fatty alcohols, nonoxynols, poloxamers, polyoxyethylenes, polyethylene glycols, dextrose, glycerol, digitonin and similar agents. *"Remington's Pharmaceutical Sciences"* by E. W. Martin describes diluents, adjuvants or excipients as suitable carriers.

The term "sustained-release" is used in the conventional sense, referring to a delivery system of a compound that provides the gradual release of that compound over a period of time and preferably, although not necessarily, with constant levels of compound release over a period of time.

Examples of delivery systems or sustained-release systems are liposomes, mixed liposomes, oleosomes, milliparticles, microparticles, nanoparticles and solid lipid nanoparticles, sponges, cyclodextrins, vesicles, micelles, mixed micelles, millispheres, microspheres, nanospheres, lipospheres, millicapsules, microcapsules and nanocapsules, as well as microemulsions and nanoemulsions, which can be added in order to achieve greater penetration of the active ingredient and/or to improve its pharmacokinetic and pharmacodynamic properties.

Sustained-release systems can be prepared using methods known in the state of the art. The amount of *Pseudoalteromonas* ferment extract contained in the sustained-release system will depend, for example, on the site of administration, kinetics and duration of release of the *Pseudoalteromonas* ferment extract, as well as the nature of the condition, disorder and/or pathology to be treated or prevented.

In another particular embodiment, the cosmetic or dermopharmaceutical compositions of the present invention can be used in different types of topical or transdermal compositions that will optionally include the cosmetically or dermopharmaceutically acceptable excipients required for the formulation of the desired form of administration ["Tratado de Farmacia Galénica" (Treaty of Galenic Pharmacy), C. Faulí ed., Luzán 5 (1993)].

The cosmetic or dermopharmaceutical compositions for topical or transdermal application of the present invention can be presented in any solid, liquid or semi-solid formulation, such as, including but not limited to, creams, multiple emulsions, such as, including but not limited to, oil in water emulsions, silicone in water emulsions, water in oil emulsions, water in silicone emulsions, water/oil/water emulsions, water/silicone/water emulsions, oil/water/oil emulsions, silicone/water/silicone emulsions, anhydrous compositions, aqueous dispersions, oils, milks, balsams, foams, lotions, gels, cream gels, hydroalcoholic solutions, hydroglycolic solutions, liniments, sera, soaps, shampoos, conditioners, serums, polysaccharide films, ointments, mousses, pomades, powders, bars, pencils, sprays or aerosols, including leave-on and rinse-off formulations. These formulations for topical or transdermal application can be incorporated using techniques known by the person skilled in the art to different types of solid accessories, such as, including but not limited to, wipes, hydrogels, adhesive patches, non-adhesive patches, microelectric patches or facial masks, or they can be incorporated to different make-up products such as make-up foundations, for example, including but not limited to, fluid foundations and compact foundations, make-up removal lotions, make-up removal milks, concealers, eye shadow, lipstick, lip protectors, lip glosses and powders, among others. They can also be incorporated to products for the treatment, care and/or cleansing of nails and cuticles, such as enamels, enamel removers and cuticle remover lotions, among others.

The cosmetic or pharmaceutical compositions of the invention may include agents that increase the percutaneous absorption of *Pseudoalteromonas* ferment extract, such as, including but not limited to, dimethylsulfoxide, dimethylacetamide, dimethylformamide, surfactants, (1-dodecyl-aza-cycloheptane-2-one) azone, alcohol, urea, ethoxydiglycol, acetone, propylene glycol or polyethylene glycol, among others.

The cosmetic or dermopharmaceutical composition of the invention can also be adsorbed on solid organic polymers or solid mineral supports, such as, including but not limited to, talcum, bentonite, silica, starch or maltodextrin, among others. Solid mineral supports can also include various organic and/or inorganic pigments.

The cosmetic or dermopharmaceutical composition of the invention can also be incorporated to fabrics, non-woven fabrics and medical devices that are in direct contact with the skin, scalp and/or nails of the body, so that they release the composition of the invention either by biodegradation of the anchoring system to the fabric, non-woven fabric or medical device or by friction of the latter with the body, by body moisture, by the pH of the skin or by body temperature. Furthermore, fabrics and non-woven fabrics can be used to prepare garments that are in direct contact with the skin, scalp and/or nails. Preferably, the fabrics, non-woven fabrics and medical devices containing the composition of the invention are used for the treatment and/or care of those conditions, disorders and/or pathologies of the skin, scalp and/or nails associated with excessive water loss and/or an alteration of the barrier function of the skin, scalp and/or nails.

Examples of fabrics, non-woven fabrics, garments and medical devices and the means to immobilize compositions to them can be found in literature and are known in the state of the art [C.K. Schaab, "Impregnating Fabrics With Microcapsules", HAPPI May 1986; G. Nelson, Int. J. Pharm., 242, 55-62 (2002); U.C. Hipler et al. "Biofunctional Textiles and the Skin" Curr. Probl. Dermatol. 2006 v.3, eds. S. Karger AG; R.K. Malcom et al., J. Cont. Release, 97, 313-320 (2004)]. Preferred fabrics, non-woven fabrics, garments and medical devices are bandages, gauzes, shirts, socks, stockings, underwear, girdles, gloves, diapers, sanitary towels, dressings, bedspreads, wipes, hydrogels, adhesive patches, non-adhesive patches, microelectric patches and/or facial masks.

In another particular embodiment, the cosmetic or dermopharmaceutical composition of the invention may contain a cosmetically or dermopharmaceutically effective amount of one of several adjuvants commonly used in compositions for the treatment and/or care of the skin, scalp and/or nails, such as, including but not limited to, skin conditioners, such as for example, cosmetic or dermopharmaceutical adjuvants that recover and/or maintain skin barrier function, anti-hyperkeratosis agents, comedolytic agents, anti-seborrheic agents, anti-psoriasis agents, epidermal hydrolytic enzymes, alpha-hydroxy acids, beta-hydroxy acids, desquamating agents, exfoliants, keratolytic agents, reactive carbonyl species scavengers, agents stimulating or inhibiting melanin synthesis, whitening or depigmenting agents, propigmentation agents, self-tanning agents, anti-aging agents, agents inhibiting NO-synthase, organic dissolvents, liquid propellants, vitamins, hormones, amino acids, anti-wrinkle agents, skin-relaxing agents, agents capable of reducing or treating bags under the eyes, anti-itching agents, agents for the treatment and/or care of sensitive skin, firming agents, astringent agents, agents stimulating lipolysis, anti-cellulite agents, anti-perspirant agents, calming agents, anti-inflammatory agents, analgesic agents, anesthetic agents, agents acting on cell metabolism, agents to improve the dermal-epidermal junction, humectants, substances that retain moisture, moisturizers, emollients, emulsifying agents, agents stimulating the synthesis of lipids and components of the stratum corneum (ceramides, fatty acids, proteins, etc.), agents inhibiting $5\alpha$-reductase, agents inhibiting lysyl- and/or prolyl-hydroxylase, antioxidant agents, free radical scavengers and/or agents against atmospheric pollution, anti-glycation agents, antimicrobial agents, fungicidal agents, fungistatic agents, bactericidal agents, bacteriostatic agents, antihistamine agents, antiemetic agents, antiviral agents, anti-parasitic agents, agents stimulating synthesis of dermal or epidermal macromolecules and/or capable of inhibiting or preventing their degradation, such as, including but not limited to, agents stimulating collagen synthesis, agents stimulating elastin synthesis, agents stimulating decorin synthesis, agents stimulating laminin synthesis, agents stimulating defensin synthesis, agents stimulating chaperone synthesis, agents stimulating sirtuin synthesis, agents stimulating hyaluronic acid synthesis, agents stimulating aquaporin synthesis, agents stimulating fibronectin synthesis, agents inhibiting matrix metalloprotease, agents inhibiting collagen degradation, agents inhibiting elastin degradation, agents inhibiting serine protease such as leukocyte elastase or cathepsin G, agents stimulating fibroblast proliferation, agents stimulating keratinocyte proliferation, agents stimulating adipocyte proliferation, agents regulating keratinocyte differentiation, agents stimulating keratinocyte differentiation, agents stimulating adipocyte differentiation, agents inhibiting acetylcholinesterase, agents inhibiting vascular permeability, venotonic agents, agents stimulating angiogenesis, agents stimulating glycosaminoglycan synthesis, DNA repairing agents, DNA protecting agents, anti-stretch mark agents, agents regulating sebum production, agents stimulating healing, coadjuvant healing agents, reepithelialization agents, cytokine growth factors, agents acting on capillary circulation and/or microcirculation, agents inducing hair growth, agents inhibiting or retarding hair growth, muscle relaxants, pigments or colorants, dyes, gelling polymers, thickeners, softeners, preservatives, stabilizing agents, perfumes, odor absorbents, chelating agents, plant extracts, essential oils, marine extracts, agents obtained from a biofermentation process, mineral salts, cell extracts and sunscreens (organic or mineral photoprotection agents active against ultraviolet A and/or B rays) among others and/or mixtures thereof, as long as they are physically and chemically compatible with the rest of the components of the composition of the present invention. The nature of these additional adjuvants can be of synthetic or natural origin, such as, for example, plant extracts, or from a biofermentation process. Additional examples can be found described in CTFA Cosmetic Ingredient Handbook, Twelfth Edition 2008.

Additionally, the cosmetic or dermopharmaceutical composition of the present invention may comprise a cosmetically or dermopharmaceutically effective amount of at least one compound, oil or wax selected from the group of cosmetic or dermopharmaceutical adjuvants consisting of humectants, substances that retain moisture, moisturizers, emollients, and mixtures thereof, such as, including but not limited to, polyols and polyesters such as glycerin, ethylhexylglycerin, caprylyl glycol, pentylene glycol, butylene glycol, propylene glycol and derivatives thereof, triethylene glycol, polyethylene glycol, Glycereth-26, Sorbeth-30; panthenol; pyroglutamic acid and salts and derivatives thereof; amino acids, such as, for example, serine, proline, alanine, glutamate or arginine; ectoine and derivatives thereof; *N*-(2-hydroxyethyl)acetamide; *N*-lauroyl-pyrrolidonecarboxylic acid; *N*-lauroyl-L-lysine; *N*-alpha-benzoyl-L-arginine; urea; creatine; alpha- and beta-hydroxy acids such as lactic acid, glycolic acid, malic acid, citric acid or salicylic acid, and salts thereof; polyglyceryl acrylate; sugars and polysaccharides, such as glucose, saccharide isomerate, sorbitol, pentaerythritol, inositol, xylitol, trehalose and derivatives thereof, sodium glucuronate, carrageenan *(Chondrus crispus)* or chitosan; glycosaminoglycans such as hyaluronic acid and derivatives thereof; aloe vera in any of its forms; honey; soluble collagen; lecithin and phosphatidylcholine; ceramides; cholesterol and esters thereof; tocopherol and esters thereof, such as tocopheryl acetate or tocopheryl linoleate; long-chain alcohols such as cetearyl alcohol, stearyl alcohol, cetyl alcohol, oleyl alcohol, isocetyl alcohol or octadecan-2-ol; long-chain alcohol esters such as lauryl lactate, myristyl lactate or alkyl benzoates $C_{12}$-$C_{15}$; fatty acids such as stearic acid, isostearic acid or palmitic acid; polyunsaturated acids (PUFAs); sorbitans such as sorbitan distearate; glycerides such as glyceryl monoricinoleate, glyceryl monostearate, glyceryl stearate citrate or triglyceride of caprylic and capric acids; sucrose esters such as sucrose palmitate or sucrose oleate; butylene glycol esters, such as dicaprylate and dicaprate; fatty acid esters such as isopropyl isostearate, isobutyl palmitate, isocetyl stearate, isopropyl laurate, hexyl laurate, decyl oleate, cetyl palmitate, di-n-butyl sebacate, isopropyl myristate, isopropyl palmate, isopropyl stearate, butyl stearate, butyl myristate, isopropyl linoleate, 2-ethylhexyl palmitate, 2-ethylhexyl cocoate, decyl oleate, myristyl myristate; squalene; mink oil; lanolin and derivatives thereof; acetylated lanolin alcohols; silicone derivatives such as cyclomethicone, dimethicone or dimethylpolysiloxane; petrolatum; mineral oil; mineral and synthetic waxes; bees wax (cera alba); paraffin; or waxes and oils of vegetable origin such as candelilla wax *(Euphorbia cerifera)*, carnauba wax *(Copernicia cerifera)*, shea butter *(Butirospermum parkii)*, cocoa butter *(Theobroma cacao)*, castor oil *(Ricinus communis)*, sunflower oil *(Helianthus annuus)*, olive oil *(Olea europaea)*, coconut oil *(Cocos nucifera)*, palm oil *(Elaeis guineensis)*, wheat germ oil *(Triticum vulgare)*, sweet almond oil *(Prunus amygdalus dulces)*, musk rose seed oil *(Rosa moschata)*, soybean oil *(Glycine soja)*, grape seed oil *(Vitis vinifera)*, calendula oil *(Calendula officinalis)*, jojoba oil *(Simmonsis chinensis)*, mango oil *(Mangifera indica)*, avocado oil *(Persea gratissima)*, and/or mixtures thereof, among others.

Preferably, the cosmetic or dermopharmaceutical adjuvants that recover and/or maintain the barrier function of the skin formed by humectants, moisture retaining substances, moisturizers, emollients, and mixtures thereof, are selected from the group consisting of glycerin, ethylhexylglycerin, caprylyl glycol, serine, proline, alanine, urea, glucose, carrageenan *(Chondrus crispus),* hyaluronic acid, phosphatyldylcholine, cetearyl alcohol, alkyl benzoates $C_{12}$-$C_{15}$, stearic acid, palmitic acid, glyceryl monostearate, glyceryl stearate citrate or triglyceride of caprylic and capric acid, butylene glycol dicaprylate/dicaprate, 2-ethylhexyl palmitate, 2-ethylhexyl cocoate, dimethicone, shea butter *(Butirospermum parkii)*, musk rose seed oil *(Rosa moschata)* and/or mixtures thereof.

In addition, the cosmetic or dermopharmaceutical composition of the invention may contain a cosmetically or dermopharmaceutically effective amount of at least one compound or extract selected from the group of keratolytic agents and/or exfoliating agents and/or desquamating agents, such as, including but not limited to, hydroxy acids and derivatives thereof; beta-hydroxy acids, particularly salicylic acid and derivatives thereof, or gentisic acid; alpha-hydroxy acids and salts thereof, such as glycolic acid, ammonium glycolate, lactic acid, 2-hydroxyoctanoic acid, alpha-hydroxycaprylic acid, mandelic acid, citric acid, malic acid or tartaric acid; alpha- and beta-hydroxybutyric acids; polyhydroxy acids, such as gluconic acid, glucuronic acid or saccharic acid; keto acids, such as pyruvic acid, glyoxylic acid; pyrrolidonecarboxylic acid; cysteic acid and derivatives; aldobionic acids; azelaic acid and derivates thereof, such as azeloyl diglycinate; ascorbic acid and derivates thereof, such as ascorbyl glucoside and sodium or magnesium ascorbyl phosphate; nicotinic acid, esters thereof and nicotinamide (also called vitamin B3 or vitamin PP); nordihydroguaiaretic acid; urea; oligofucoses; cinnamic acid; jasmonic acid derivatives; hydroxystilbenes, such as resveratrol; *Saccarum officinarum* extract; enzymes involved in the desquamation and degradation of corneodesmosomes, such as, for example, glycosidases, stratum corneum chymotryptic enzyme (SCCE) or other proteases such as trypsin, chemotrypsin, sutilains, papain or bromelain; chelating agents, such as ethylenediaminetetraacetic acid (EDTA), aminosulfonic compounds such as 4-(2-hydroxyethyl)piperazine-1-ethanesulfonic acid (HEPES) or sodium methylglycine diacetic acid (TRILON® M marketed by BASF); derivatives of 2-oxothiazolidine-4-carboxylic acid (procysteine); sugar derivatives such as O-octanoyl-6-D-maltose and N-acetylglucosamine; chestnut extract *(Castanea sativa)* such as the one marketed by SILAB under the name Recoverine® [INCI: Water (Aqua), Castanea Sativa Seed Extract]; opuntia extract *(Opuntia ficus-indica)* such as the one marketed by SILAB as Exfolactive® [INCI: Hydrolyzed Opuntia Ficus Indica Flower Extract]; or Phytosphingosine SLC® [INCI: Salicyloyl Phytosphingosine] marketed by Degussa/Evonik; extract or combination of extracts of *Saphora japonica*, papaya, pineapple, squash or sweet potato, and/or mixtures thereof. Preferably, the keratolytic agents and/or exfoliating agents and/or desquamating agents are selected from the group consisting of salicylic acid, ammonium glycolate, lactic acid, malic acid, citric acid, urea, resveratrol, EDTA, papain, bromelain, or an extract or combination of extracts of *Saphora japonica*, papaya or pineapple and/or mixtures thereof. More preferably, they are selected from the group consisting of urea and EDTA.

The cosmetic or dermopharmaceutical composition of the invention may contain a cosmetically or dermopharmaceutically effective amount of at least one compound or extract selected from the group of agents stimulating or regulating keratinocyte differentiation, such as, including but not limited to, minerals such as calcium, retinoids such as retinol or trenitoin, vitamin D3 analogs such as calcitriol, calcipotriol or tacalcitol, lupin extract *(Lupinus albus)* such as the one marketed by SILAB under the name Structurin® [INCI: Hydrolyzed Lupine Protein], beta-sitosterol sulfate, such as the one marketed by Vincience/ISP under the name Phytocohesine PSP® [INCI: Sodium Beta-sitosterol Sulfate], corn seed extract *(Zea Mays)* such as the one marketed by Solabia with the name Phytovityl C® [INCI: Water (Aqua), Zea Mays Extract], *Helix Aspersa Müller* glycoconjugates and/or mixtures thereof.

In addition, the cosmetic or dermopharmaceutical compositions of the present invention may additionally contain a cosmetically or dermopharmaceutically effective amount of at least one compound or extract or oil with analgesic and/or anti-inflammatory activity. As for example, including but not limited to, the analgesic and/or anti-inflammatory agents are selected from the group consisting of hydrocortisone, clobetasol, dexamethasone, prednisone, paracetamol, acetylsalicylic acid, amoxiprin, benorylate, choline salicylate, diflunisal, faislamine, methyl salicylate, magnesium salicylate, salsalate, diclofenac, aceclofenac, acemetacin, bromfenac, etodolac, indometacin, sulindac, tolmetin, ibuprofen, carprofen, fenbufen, fenoprofen, flurbiprofen, ketoprofen, ketorolac, loxoprofen, naproxen, oxaprozin, tiaprofenic acid, suprofen, mefenamic acid, meclofenamate, meclofenamic acid, nabumetone, phenylbutazone, azapropazone, metamizole, oxyphenbutazone, sulfinpyrazone, piroxicam, lornoxicam, meloxicam, tenoxicam, celecoxib, etoricoxib, lumiracoxib, parecoxib, rofecoxib, valdecoxib, nimesulide, licofelone, morphine, codeine, oxycodone, hydrocodone, diamorphine, pethidine, tramadol, buprenorphine, benzocaine, lidocaine, chloroprocaine, tetracaine, procaine, tricyclic antidepressants, amitriptyline, carbamazepine, gabapentine, pregabaline, bisabolol, panthenol, biotin, disodium lauriminodipropionate tocopheryl phosphate, ciclopirox olamine, nordihydroguaiaretic acid, Neutrazen™ [INCI: Water (Aqua), Butylenen Glycol, Dextran, Palmitoyl Tetrapeptide-8] marketed by Atrium Innovations/Unipex Group, Meliprene® [INCI: Dextran, Acetyl Heptapeptide-1] marketed by Institut Européen de Biologie Cellulaire/Unipex Group, coenzyme Q10 or alkylglycerine ethers, and/or mixtures thereof, or natural extracts or essential oils with intrinsic analgesic and/or anti-inflammatory activity, such as, including but not limited to, madecassoside, echinacea, amaranth seed oil, sandalwood oil, peach tree leaf extract, *Aloe vera*, *Arnica montana*, *Artemisia vulgaris*, *Asarum maximum*, *Calendula officinalis, Capsicum, Centipeda cunninghamii, Chamomilla recutita, Crinum asiaticum, Hamamelis virginiana, Harpagophytum procumbens, Hypericum perforatum, Lilium candidum, Malva sylvestris, Melaleuca alternifolia, Origanum majorana, Salix alba, Silybum marianum, Tanacetum parthenium* or *Uncaria guianensis* extract and/or mixtures thereof, among others.

The cosmetic or dermopharmaceutical composition of the invention may also additionally contain a cosmetic or dermopharmaceutically effective amount of at least one bactericidal and/or bacteriostatic agent and a fungicidal agent and/or or a fungistatic agent, such as, including but not limited to, caprylyl glycol, imidazolidinyl urea, methyl 4-hydroxybenzoate [INCI: methylparaben], ethyl 4-hydroxybenzoate [INCI: ethylparaben], propyl 4-hydroxybenzoate [INCI: propylparaben], butyl 4-hydroxybenzoate [INCI: butylparaben], isobutyl 4-hydroxybenzoate [INCI: isobutylparaben], 1,3-bis(hydroxymethyl)-5,5-dimethylimidazolidine-2,4-dione [INCI: DMDM Hydantoin], benzyl 4-hydroxybenzoate [INCI: benzylparaben], benzyl alcohol, dehydroacetic acid, benzoic acid, sorbic acid, salicylic acid, formic acid, propionic acid, 2-bromo-2-nitropropan-1,3-diol, 3-p-chlorophenoxy-1,2-propanediol [INCI: chlorphenesin], dichlorobenzyl alcohol, iodopropynyl butylcarbamate, benzalkonium chloride, benzethonium chloride, chlorhexidine, ethanol, isopropanol, methanol, 1,2-hexanediol, 1,2-octanediol, pentylene glycole, glyceryl laurate, glyceryl caprylate, glyceryl caprate, benzoyl peroxide, chlorhexidine gluconate, triclosan, phenoxyethanol, terpinene-4-ol, $\alpha$-terpineol, resorcinol, stiemycin, erythromycin, neomycin, clindamycin and esters thereof, tetracyclines, metronidazole, azelaic acid, tolnaftate, nystatin, clotrimazole, ketoconazole, zinc pyrithione, zinc oxide, isothiazolinones, selenium sulfide, benzylhemiformal, boric acid, sodium borate, 6,6-dibromo-4,4-dichloro-2,2'-methylenediphenol [INCI: bromochlorophene], 5-bromo-5-nitro-1,3-dioxane, sodium tosylchloramide [INCI: chloramine T], chloroacetamide, p-chloro-m-cresol, 2-benzyl-4 chlorophenol [INCI: chlorophene], dimethyl oxazolidine, dodecyldimethyl-2-phenoxyethyl ammonium bromide [INCI: domiphen bromide], 7-ethylbicyclooxazolidine, glutaraldehyde, *N*-(4-chlorophenyl)-*N*-[4-chloro-3-(trifluoromethyl)phenyl]-urea [INCI: cloflucarban], hexetidine, 2-hydroxy-4-isopropyl-2,4,6-cycloheptatrien-1-one [INCI: Hinokitiol], isopropylmethylphenol, mercury salts, aluminum salts, nysine, phenoxyisopropanol, o-phenylphenol, 3-heptyl-2-[(3-heptyl-4-methyl-3H-thiazol-2-ilyden)methyl]-4-methylthiazolium iodide [INCI: Quaternium-73], silver chloride, sodium iodate, thymol, undecylenic acid, diethylenetriaminepentaacetic acid, ethylenediaminetetraacetic acid, lactoperoxidase, glucose oxidase, lactoferrin, and mixtures thereof, and/or a cosmetically or pharmaceutically effective amount of at least one natural extract or essential oil with bactericidal and/or bacteriostatic activity, and/or with intrinsic fungicidal and/or fungistatic activity, such as, including but not limited to *Allium sativum, Calendula officinalis, Chamomilla recutita, Echinacea Purpura, Hyssopus Officinalis, Melaleuca alternifolia* extracts or tea tree oil and/or mixtures thereof, among others. Preferably, the bactericidal and/or bacteriostatic and/or fungicidal and/or fungistatic cosmetic or dermapharmaceutical agents are selected from the group consisting of imidazolidinyl urea, methyl 4-hydroxybenzoate [INCI: methylparaben], ethyl 4-hydroxybenzoate INCI: ethylparaben], propyl 4-hydroxybenzoate [INCI: propylparaben], butyl 4-hydroxybenzoate [INCI: butylparaben], isobutyl 4-hydroxybenzoate [INCI: isobutylparaben], 1,3-bis(hydroxymethyl)-5,5-dimethylmidazolidine-2,4-dione [INCI: DMDM Hydantoin], benzyl 4-hydroxybenzoate [INCI: benzylparaben], benzyl alcohol, benzyl peroxide or ethylenediaminetetraacetic acid, extracts of *Calendula officinalis, Chamomilla recutita*, and mixtures thereof, among others. More preferably, they are selected from the group consisting of imidazolidinyl urea, methyl 4-hydroxybenzoate [INCI: methylparaben], ethyl 4-hydroxybenzoate [INCI: ethylparaben], propyl 4-hydroxybenzoate [INCI: propylparaben], butyl 4-hydroxybenzoate [INCI: butylparaben], isobutyl 4-hydroxybenzoate [INCI: isobutylparaben] or ethylenediaminetetraacetic acid and/or mixtures thereof.

The cosmetic or dermopharmaceutical compositions of the present invention may additionally contain a cosmetically or dermopharmaceutically effective amount of at least one antiviral, such as, including but not limited to, aciclovir, docosanol or tromantadine, and/or an anti-parasitic agent, such as, including but not limited to, metronidazole, crotamiton or pyrethroids.

The cosmetic or dermopharmaceutical compositions of the present invention may additionally contain a cosmetically or dermopharmaceutically effective amount of at least one anti-itch agent, such as, including but not limited to, thenalidine, trimeprazine or cyproheptadine.

Additionally, the cosmetic or dermopharmaceutical composition of the present invention may contain a cosmetically or dermopharmaceutically effective amount of at least one extract or combination or extracts of agents stimulating healing, coadjuvant healing agents and/or reepithelialization agents, such as, including but not limited to, extracts of *Aristoloquia clematis, Centella asiatica, Rosa moschata, Echinacea angustifolia, Symphytum officinal, Equisetum arvense, Hypericum perforatum, Mimosa tenuiflora, Persea gratísima, Prunus africanum, Tormentilla erecta, Aloe vera,* Polyplant® Epithelizing [INCI: Calendula Officinalis, Hypericum Perforatum, Chamomilla Recutita, Rosmarinus Officinalis] marketed by Provital, Cytokinol® LS 9028 [INCI: Hydrolyzed Casein, Hydrolyzed Yeast Protein, Lysine HCl] marketed by Laboratories Sérobiologiques/Cognis or Deliner® [INCI: Zea Mays (Corn) Kernel Extract] marketed by Coletica/Engelhard among others, and/or additionally a cosmetically or dermopharmaceutically effective amount of at least one synthetic compound, extract or product from a biofermentation procedure that is a agent stimulating healing, coadjuvant healing agent and/or reepithelialization agent, such as, including but not limited to, allantoin, cadherins, integrins, selectins, hyaluronic acid receptors, immunoglobulins, fibroblast growth factor, connective tissue growth factor, platelet derived growth factor, vascular endothelial growth factor, epidermal growth factor, insulin-like growth factor, keratinocyte growth factor, colony stimulating factors, transforming growth factors-beta, tumor necrosis factor-alpha, interferons, interleukins, matrix metalloproteases, protein tyrosine phosphatase receptors, or Decorinyl™ [INCI: Tripeptide-10 Citrulline], marketed by Lipotec, among others. Preferably, the extracts or combinations of extracts of agents stimulating healing, coadjuvant healing agents and/or reepithelialization agents are selected from the group consisting of *Rosa moschata, Hypericum perforatum, Aloe vera,* allantoin and/or mixtures thereof, among others.

The cosmetic or dermopharmaceutical composition of the present invention can be a final composition, available for its application without having to perform any concentration, solution, dilution, dispersion, pulverization, spray process or any other similar process known by the person skilled in the art, or an intermediate composition to which one or several of the processes above will be performed or any other process known by the person skilled in the art in order to obtain a final composition.

The following specific examples provided here illustrate the nature of the present invention. These examples are included for illustrative purposes only and should not be understood as limitations to the invention claimed herein.

In a second aspect, the present invention refers to the use of *Pseudoalteromonas* ferment extract in the preparation of the cosmetic or dermopharmaceutical composition of the present invention for the treatment and/or care of the conditions, disorders and or pathologies associated with an alteration of the barrier function of the skin, scalp and/or nails, and preferably to an alteration of the keratinization.

In a third aspect, the present invention refers to a method for the treatment and/or care of the conditions, disorders and/or pathologies associated with an alteration of the barrier function of the skin, scalp and/or nails, preferably to an alteration of the keratinization, which comprises the topical or transdermal application on the skin, scalp and/or nails of the cosmetic or dermopharmaceutical composition of the present invention.

## EXAMPLES

General methodology for the moisturization tests

*Method of evaluation*

Measurements of skin moisturization before and after treatment were used to evaluate the results of applying different compositions.

Volunteers were selected from among men and women between the ages of 34 and 68 wothout pathologies immediately before or during the study, who were also not taking any medication that could affect the results and who had no history of intolerance to medications and/or cosmetic products. During the study period, subjects did not use other products on the areas being tested and avoided exposure to UV radiation

*Instrumentation*

The instrumental measurement of skin moisturization was done using a Courage&Khazaka CM 825 corneometer. The values obtained, directly proportional to the amount of water contained in the stratum corneum and that represented the moisturization level of the skin surface, are expressed in corneometric units (c.u.). The device can measure values ranging between 0 and 150 c.u.

The study was carried out in a bioclimatic room (24 $\pm$ 2°C; 50 $\pm$ 10% ambient humidity) with the purpose of maintaining temperature and humidity constant during measurements.

*Mathematical processing*

The results were statistically processed. For each measurement, the mean value of 3 corneometry readings taken at contiguous points of the same area were used. Mean values and standard deviations were calculated for the instrumental initial and final moisturization values. Also calculated was

$$T_f - T_0 = \text{parameter variation}$$

where $T_f$= mean value at the end of treatment and $T_0$= mean value at the beginning of treatment. This difference is also shown in the results as percentage variation.

The initial and final values were compared by applying a paired Student's t-test. Differences between initial and final values were considered significant when probability p was $\leq 0.05$.

EXAMPLE 1

*Composition of a hand and foot cream*

| INGREDIENT (INCI Nomenclature) | % IN WEIGHT |
|---|---|
| **ANTARCTICINE C** (PSEUDOALTEROMONAS FERMENT EXTRACT) | 1.00 |
| CARRAGEENAN (CHONDRUS CRISPUS) | 0.07 |
| GLUCOSE | $3.5 \cdot 10^{-3}$ |
| XANTHAN GUM | 0.12 |
| GLYCERIN | 5.00 |
| UREA | 3.00 |
| MUSK ROSE (ROSA MOSCHATA) SEED OIL, ASCORBYL PALMITATE | 0.20 |
| SHEA BUTTER (BUTYROSPERMUM PARKII) | 5.00 |
| ETHYLHEXYL PALMITATE | 7.00 |
| C12-15 ALKYL BENZOATE | 2.50 |
| CAPRYLIC/CAPRIC TRIGLYCERIDE | 5.00 |
| STEARIC ACID, PALMITIC ACID | 3.00 |
| GLYCERYL STEARATE | 4.50 |
| GLYCERYL STEARATE CITRATE | 1.00 |
| POLYGLYCERYL-3 STEARATE | 3.00 |
| BUTYLEN GLYCOL DICAPRYLATE/DICAPRATE | 2.00 |
| DIMETHICONE | 2.00 |
| CARBOMER | 0.30 |
| DISODIUM EDTA | 0.30 |
| PHENOXYETHANOL, METHYLPARABEN, ETHYLPARABEN, BUTYLPARABEN, PROPYLPARABEN, ISOBUTYLPARABEN | 0.88 |
| IMIDAZOLIDINYL UREA | 0.20 |
| BHT | 0.05 |
| FRAGRANCE (PARFUM) | 0.20 |
| WATER (AQUA) | q.s. |

EXAMPLE 2

*Intermediate composition*

| INGREDIENT (INCI Nomenclature) | % IN WEIGHT |
|---|---|
| **ANTARCTICINE C** (PSEUDOALTEROMONAS FERMENT EXTRACT) | 15.00 |
| CAPRYLYL GLYCOL | 0.90 |
| ETHYLHEXYLGLYCERIN | 0.70 |
| L-SERINE, L-ALANINE, L-PROLINE | 0.30 |
| XANTHAN GUM | 1.70 |
| GLYCERIN | 25.00 |
| WATER (AQUA) | q.s.p. 100 |

EXAMPLE 3

*Composition of a hand and foot cream*

| INGREDIENT (INCI Nomenclature) | % IN WEIGHT |
|---|---|
| **ANTARCTICINE C** (PSEUDOALTEROMONAS FERMENT EXTRACT) | 1.05 |
| CAPRYLYL GLYCOL | 0.06 |
| ETHYLHEXYLGLYCERIN | 0.05 |
| L-SERINE, L-ALANINE, L-PROLINE | 0.02 |
| XANTHAN GUM | 0.12 |
| GLYCERIN | 6.75 |
| UREA | 3.00 |
| MUSK ROSE (ROSA MOSCHATA) SEED OIL, ASCORBYL PALMITATE | 0.20 |
| SHEA BUTTER (BUTYROSPERMUM PARKII) | 5.00 |
| ETHYLHEXYL PALMITATE | 7.00 |
| C12-15 ALKYL BENZOATE | 2.50 |
| CAPRYLIC/CAPRIC TRIGLYCERIDE | 5.00 |
| STEARIC ACID, PALMITIC ACID | 3.00 |
| GLYCERYLSTEARATE | 4.50 |
| GLYCERYL STEARATE CITRATE | 1.00 |
| POLYGLYCERYL-3 STEARATE | 3.00 |
| BUTYLEN GLYCOL DICAPRYLATE/DICAPRATE | 2.00 |
| DIMETHICONE | 2.00 |
| CARBOMER | 0.30 |
| DISODIUM EDTA | 0.30 |
| PHENOXYETHANOL, METHYLPARABEN, ETHYLPARABEN, BUTYLPARABEN, PROPYLPARABEN, ISOBUTYLPARABEN | 0.88 |
| IMIDAZOLIDINYL UREA | 0.20 |
| BHT | 0.05 |
| FRAGRANCE (PARFUM) | 0.20 |
| WATER (AQUA) | q.s.p. 100 |

EXAMPLE 4

*Composition of a gel*

| INGREDIENT (INCI Nomenclature) | % IN WEIGHT |
|---|---|
| **ANTARCTICINE C** (PSEUDOALTEROMONAS FERMENT EXTRACT) | 1.05 |

(continued)

| INGREDIENT (INCI Nomenclature) | % IN WEIGHT |
|---|---|
| CAPRYLYL GLYCOL | 0.06 |
| ETHYLHEXYLGLYCERIN | 0.05 |
| L-SERINE, L-ALANINE, L-PROLINE | 0.02 |
| GLYCERIN | 1.75 |
| XANTHAN GUM | 0.12 |
| HYALURONIC ACID | 0.008 |
| PHOSPHATIDYLCHOLINE | 0.4 |
| WATER (AQUA), GLYCERIN, GLYCERYL POLYACRYLATE | 20.00 |
| DISODIUM EDTA | 0.15 |
| PHENOXYETHANOL, METHYLPARABEN, ETHYLPARABEN, BUTYLPARABEN, PROPYLPARABEN, ISOBUTYLPARABEN | 0.50 |
| IMIDAZOLIDINYL UREA | 0.20 |
| FRAGRANCE (PARFUM) | 0.15 |
| WATER (AQUA) | q.s.p. 100 |

EXAMPLE 5

*Composition of a face cream*

| INGREDIENT (INCI Nomenclature) | % IN WEIGHT |
|---|---|
| **ANTARCTICINE C** (PSEUDOALTEROMONAS FERMENT EXTRACT) | 1.05 |
| CAPRYLYL GLYCOL | 0.06 |
| ETHYLHEXYLGLYCERIN | 0.05 |
| L-SERINE, L-ALANINE, L-PROLINE | 0.02 |
| GLYCERIN | 1.75 |
| XANTHAN GUM | 0.12 |
| ETHYLHEXYL COCOATE | 2.50 |
| C12-15 ALKYL BENZOATE | 5.00 |
| CETEARYL ALCOHOL, CETEARYL GLUCOSIDE | 5.00 |
| WATER (AQUA), POLYACRYLAMIDE, C13-14 ISOPARAFFIN, LAURETH-7 | 2.00 |
| POTASSIUM CETYL PHOSPHATE | 0.50 |
| DIMETHICONE | 1.00 |
| MENTHYL LACTATE | 0.3 |
| TOCOPHERYL ACETATE | 0.5 |
| DISODIUM EDTA | 0.30 |
| PHENOXYETHANOL, METHYLPARABEN, ETHYLPARABEN, BUTYLPARABEN, PROPYLPARABEN, ISOBUTYLPARABEN | 0.85 |
| IMIDAZOLIDINYL UREA | 0.20 |
| CI 42090 (BLUE 1) | 0.02 |
| FRAGRANCE (PARFUM) | 0.30 |
| WATER (AQUA) | q.s.p. 100 |

EXAMPLE 6

*Treatment of plantar hyperkeratosis*

To evaluate the efficacy of the composition from Example 3 for plantar hyperkeratosis treatment, the cream was applied to the feet of 20 Caucasian volunteers between the ages of 39 and 68 (mean age, 55 years) who were suffering from plantar hyperkeratosis, twice a day, for two weeks.

The results obtained for skin moisturization in the treatment of plantar hyperkeratosis are shown in table 1.

Table 1

| $T_0$ | $T_f$ | Mean variation | % Variation | Student-t |
|-------|-------|----------------|-------------|-----------|
| 8.0 | 15.1 | 7.1 | 88.8% | $p < 0.001$ |

A mean increase of 88.8% on moisturization value was obtained in the skin of the heels of the volunteers at the end of treatment.

EXAMPLE 7

*Treatment of xerosis*

To evaluate the efficacy of the composition from Example 5 for the treatment of xerosis, the cream was applied to the faces of 20 Caucasian volunteers between the ages of 34 and 66 (mean age, 47 years) who were suffering from xerosis, twice a day, for two weeks.

The results obtained for skin moisturization in the treatment of facial xerosis are shown in table 2.

Table 2

| $T_0$ | $T_f$ | Mean variation | % Variation | Student-t |
|-------|-------|----------------|-------------|-----------|
| 31.0 | 48.2 | 17.2 | 55.7% | $p < 0.001$ |

A mean increase of 55.7% on moisturization value was obtained in the skin of the face of the volunteers.

**Claims**

1. Use of a cosmetic composition containing *Pseudoalteromonas* ferment extract [INCI: *Pseudoalteromonas* Ferment Extract] for the non-therapeutic treatment of conditions of the skin, scalp and/or nails associated with hyperkeratosis, in which said conditions are selected from the group consisting of palmar and plantar hyperkeratosis, calluses or corns, dandruff and xerosis.

2. The use according to claim 1, in which the *Pseudoalteromonas* ferment extract is added to compositions by aqueous solution, or solubilized in conventional cosmetically acceptable solvents selected from the group consisting of ethanol, propanol, isopropanol, propylene glycol, glycerin, butylene glycol, polyethylene glycol and mixtures thereof.

3. The use according to any one of claims 1 to 2, in which said cosmetic composition comprises liposomes, mixed liposomes, oleosomes, milliparticles, microparticles, nanoparticles and solid lipid nanoparticles, sponges, cyclodextrins, vesicles, micelles, mixed micelles, millispheres, microspheres, nanospheres, lipospheres, millicapsules, microcapsules, nanocapsules, microemulsions and nanoemulsions.

4. The use according to any one of claims 1 to 3, in which said cosmetic composition is presented as a formulation selected from the group consisting of creams, oil in water emulsions, silicone in water emulsions, water in oil emulsions, water in silicone emulsions, water/oil/water emulsions, water/silicone/water emulsions, oil/water/oil emulsions, silicone/water/silicone emulsions, anhydrous compositions, aqueous dispersions, oils, milks, balsams, foams, lotions, gels, cream gels, hydroalcoholic solutions, hydroglycolic solutions, liniments, sera, soaps, shampoos, conditioners, serums, polysaccharide films, ointments, mousses, pomades, powders, bars, pencils, sprays or aerosols, leave-on or rinse off formulations.

5. The use according to any one of claims 1 to 4, in which said cosmetic composition additionally comprises a cosmetically effective amount of at least one cosmetic adjuvant selected from the group consisting of epidermal hydrolytic enzymes, alpha-hydroxy acids, beta-hydroxy acids, organic solvents, liquid propellants, vitamins, hormones, amino acids, moisturizers, emollients, muscle relaxants, pigments or colorants, dyes, gelling polymers, thickeners, softeners, preservatives, perfumes, odor absorbents, essential oils, mineral salts and sunscreens, and mixtures thereof.

6. the use according to any one of claims 1 to 4, in which said cosmetic composition additionally comprises a cosmetically effective amount of at least one cosmetic adjuvant selected from the group consisting of polyols, polyethers, glycerin, ethylhexylglycerin, caprylyl glycol, pentylene glycol, butylene glycol, propylene glycol, triethylene glycol, polyethylene glycol, Glycereth-26, Sorbeth-30, panthenol, pyroglutamic acid and salts, amino acids, serine, proline, alanine, glutamate or arginine, ectoine, *N-(2-hydroxyethyl)acetamide*, *N*-lauroyl-pyrrolidonecarboxylic acid, *N*-lauroyl-L-lysine, *N*-alpha-benzoyl-L-arginine, urea, creatine, alpha-hydroxy acids and beta-hydroxy acids and salts thereof, lactic acid, glycolic acid, malic acid, citric acid, salicylic acid, polyglyceryl acrylate, sugars and polysaccharides, glucose, saccharide isomerate, sorbitol, pentaerythritol, inositol, xylitol, trehalose, sodium glucuronate, carrageenan *(Chondrus crispus),* chitosan, glycosaminoglycans, hyaluronic acid, aloe vera, honey, soluble collagen, lecithin and phosphatidylcholine, ceramides, cholesterol and esters thereof, tocopherol and esters thereof, tocopheryl acetate, tocopheryl linoleate, long-chain alcohols, cetearyl alcohol, stearyl alcohol, cetyl alcohol, oleyl alcohol, isocetyl alcohol, octadecan-2-ol, long-chain alcohol esters, lauryl lactate, myristyl lactate, alkyl benzoates $C_{12}$-$C_{15}$, fatty acids, stearic acid, isostearic acid, palmitic acid, polyunsaturated acids, sorbitans, sorbitan distearate, glycerides, glyceryl monoricinoleate, glyceryl monostearate, glyceryl stearate citrate or triglyceride of caprylic and capric acids, sucrose esters, sucrose palmitate or sucrose oleate, butyleneglycol esters, fatty acid esters, isopropyl isostearate, isobutyl palmitate, isocetyl stearate, isopropyl laurate, hexyl laurate, decyl oleate, cetyl palmitate, di-n-butyl sebacate, isopropyl myristate, isopropyl palmate, isopropyl stearate, butyl stearate, butyl myristate, isopropyl linoleate, 2-ethylhexyl palmitate, 2-ethylhexyl cocoate, decyl oleate, myristyl myristate, squalene, mink oil, lanolin, acetylated lanolin alcohols, cyclomethicone, dimethicone or dimethylpolysiloxane, petrolatum, mineral oil, mineral and synthetic waxes, bees wax, paraffin, candelilla wax, carnauba wax, shea butter, cocoa butter, castor oil, sunflower oil, olive oil, coconut oil, palm oil, wheat germ oil, sweet almond oil, musk rose seed oil, soybean oil, grape seed oil, calendula oil, jojoba oil, mango oil and avocado oil.

7. The use according to any one of claims 1 to 4, in which said cosmetic composition additionally comprises a cosmetically effective amount of at least one cosmetic adjuvant selected from the group consisting of hydroxy acids, beta-hydroxy acids, salicylic acid, gentisic acid, alpha-hydroxy acids and salts thereof, glycolic acid, ammonium glycolate, lactic acid, 2-hydroxyoctanoic acid, alpha-hydroxycaprylic acid, mandelic acid, citric acid, malic acid, tartaric acid, alpha- and beta-hydroxybutyric acids, polyhydroxy acids, gluconic acid, glucuronic acid, saccharic acid, keto acids, pyruvic acid, glyoxylic acid, pyrrolidone carboxylic acid, cysteic acid, aldobionic acids, azelaic acid, azeloyl diglycinate, ascorbic acid, ascorbyl glucoside, sodium or magnesium ascorbyl phosphate, nicotinic acid and esters thereof, nicotinamide, nordihydroguaiaretic acid, urea, oligofucoses, cinnamic acid, hydroxystilbenes, resveratrol, glycosidases, stratum corneum chymotryptic enzyme, trypsin, chemotrypsin, sutilains, papain, bromelain, chelating agents, ethylenediaminetetraacetic acid, aminosulfonic chelating compounds, 4-(2-hydroxyethyl)piperazine-1-ethanesulfonic acid, sodium methylglycine diacetic acid, O-octanoyl-6-D-maltose, *N*-acetylglucosamine, Salicyloyl Phytosphingosine, papaya, pineapple, squash and sweet potato.

8. The use according to any one of claims 1 to 4, in which said cosmetic composition additionally comprises a cosmetically effective amount of at least one cosmetic adjuvant selected from the group consisting of calcium, retinol, trenitoin, calcitriol, calcipotriol, tacalcitol, beta-sitosterol sulfate and *Helix Aspersa Müller* glycoconjugates.

9. The use according to any one of claims 1 to 4, in which said cosmetic composition additionally comprises a cosmetically effective amount of at least one cosmetic adjuvant selected from the group consisting of allantoin, cadherins, integrins, selectins, hyaluronic acid receptors, immunoglobulins, fibroblast growth factor, connective tissue growth factor, platelet derived growth factor, vascular endothelial growth factor, epidermal growth factor, insulin-like growth factor, keratinocyte growth factor, colony stimulating factors, transforming growth factors-beta, tumor necrosis factor-alpha, interferons, interleukins, matrix metalloproteases, protein tyrosine phosphatase receptors, Tripeptide-10 Citrulline, *Centella asiatica, Rosa moschata, Echinaa angustifolia, Symphytum officinal, Equisetum arvense, Hypericum perforatum, Mimosa tenuiflora, Persea gratisima, Prunus africanum, Tormentilla erecta, Aloe vera,* and mixtures thereof.

10. A dermopharmaceutical composition containing *Pseudoalteromonas* ferment extract for use in the treatment of pathologies of the skin, scalp and/or nails associated with hyperkeratinization, in which said pathologies are selected from the group consisting of reactive hyperkeratosis, actinic keratosis, non-actinic keratosis, acne, papillomas or warts, seborrheic keratosis, psoriasis, parakeratosis, pityriasis, lichen planus, Darier's disease, onychomycosis and dermatomycosis.

11. Dermopharmaceutical composition for use according to claim 10, in which *Pseudoalteromonas* ferment extract is added to compositions by aqueous solution, or solubilized in conventional dermopharmaceutically acceptable solvents selected from the group consisting of ethanol, propanol, isopropanol, propylene glycol, glycerin, butylene glycol, polyethylene glycol and mixtures thereof.

12. Dermopharmaceutical composition for use according to any one of claims 10 to 11, comprising liposomes, mixed liposomes, oleosomes, milliparticles, microparticles, nanoparticles and solid lipid nanoparticles, sponges, cyclodextrins, vesicles, micelles, mixed micelles, millispheres, microspheres, nanospheres, liposomes, millicapsules, microcapsules, nanocapsules, microemulsions and nanoemulsions.

13. Dermopharmaceutical composition for use according to any one of claims 10 to 12, that is presented as a formulation selected from the group consisting of creams, oil in water emulsions, silicone in water emulsions, water in oil emulsions, water in silicone emulsions, water/oil/water emulsions, water/silicone/water emulsions, oil/water/oil emulsions, silicone/water/silicone emulsions, anhydrous compositions, aqueous dispersions, oils, milks, balsams, foams, lotions, gels, cream gels, hydroalcoholic solutions, hydroglycolic solutions, liniments, sera, soaps, shampoos, conditioners, serums, polysaccharide films, ointments, mousses, pomades, powders, bars, pencils, sprays or aerosols, leave-on or rinse off formulations.

14. Dermopharmaceutical composition for use according to any one of claims 10 to 12, additionally comprising a dermopharmaceutically effective amount of at least one dermopharmaceutical adjuvant selected from the group consisting of epidermal hydrolytic enzymes, alpha-hydroxy acids, beta-hydroxy acids, organic solvents, liquid propellants, vitamins, hormones, amino acids, moisturizers, emollients, muscle relaxants, pigments or colorants, dyes, gelling polymers, thickeners, softeners, preservatives, perfumes, odor absorbents, essential oils, mineral salts, and sunscreens, and mixtures thereof.

15. Dermopharmaceutical composition for use according to any one of claims 10 to 12, additionally comprising a dermopharmaceutically effective amount of at least one dermopharmaceutical adjuvant selected from the group consisting of polyols, polyethers, glycerin, ethylhexylglycerin, caprylyl glycol, pentylene glycol, butylene glycol, propylene glycol, triethylene glycol, polyethylene glycol, Glycereth-26, Sorbeth-30, panthenol, pyroglutamic acid and salts, amino acids, serine, proline, alanine, glutamate or arginine, ectoine, *N-(2*-hydroxyethyl)acetamide, *N*-lauroyl-pyrrolidonecarboxylic acid, *N*-lauroyl-L-lysine, *N*-alpha-benzoyl-L-arginine, urea, creatine, alpha-hydroxy acids and beta-hydroxy acids and salts thereof, lactic acid, glycolic acid, malic acid, citric acid, salicylic acid, polyglyceryl acrylate, sugars and polysaccharides, glucose, saccharide isomerate, sorbitol, pentaerythritol, inositol, xylitol, trehalose, sodium glucuronate, carrageenan *(Chondrus crispus),* chitosan, glycosaminoglycans, hyaluronic acid, aloe vera, honey, soluble collagen, lecithin and phosphatidylcholine, ceramides, cholesterol and esters thereof, tocopherol and esters thereof, tocopheryl acetate, tocopheryl linoleate, long-chain alcohols, cetearyl alcohol, stearyl alcohol, cetyl alcohol, oleyl alcohol, isocetyl alcohol, octadecan-2-ol, long-chain alcohol esters, lauryl lactate, myristyl lactate, alkyl benzoates $C_{12}$-$C_{15}$, fatty acids, stearic acid, isostearic acid, palmitic acid, polyunsaturated acids, sorbitans, sorbitan distearate, glycerides, glyceryl monoricinoleate, glyceryl monostearate, glyceryl stearate citrate or triglyceride of caprylic and capric acids, sucrose esters, sucrose palmitate or sucrose oleate, butyleneglycol esters, fatty acid esters, isopropyl isostearate, isobutyl palmitate, isocetyl stearate, isopropyl laurate, hexyl laurate, decyl oleate, cetyl palmitate, di-*n*-butyl sebacate, isopropyl myristate, isopropyl palmate, isopropyl stearate, butyl stearate, butyl myristate, isopropyl linoleate, 2-ethylhexyl palmitate, 2-ethylhexyl cocoate, decyl oleate, myristyl myristate, squalene, mink oil, lanolin, acetylated lanolin alcohols, cyclomethicone, dimethicone or dimethylpolysiloxane, petrolatum, mineral oil, mineral and synthetic waxes, bees wax, paraffin, candelilla wax, carnauba wax, shea butter, cocoa butter, castor oil, sunflower oil, olive oil, coconut oil, palm oil, wheat germ oil, sweet almond oil, musk rose seed oil, soybean oil, grape seed oil, calendula oil, jojoba oil, mango oil and avocado oil.

16. Dermopharmaceutical composition for use according to any one of claims 10 to 12, additionally comprising a dermopharmaceutically effective amount of at least one dermopharmaceutical adjuvant selected from the group consisting of hydroxy acids, beta-hydroxy acids, salicylic acid, gentisic acid, alpha-hydroxy acids and salts thereof, glycolic acid, ammonium glycolate, lactic acid, 2-hydroxyoctanoic acid, alpha-hydroxycaprylic acid, mandelic acid, citric acid, malic acid, tartaric acid, alpha- and beta-hydroxybutyric acids, polyhydroxy acids, gluconic acid, glucuronic acid, saccharic acid, keto acids, pyruvic acid, glyoxylic acid, pyrrolidone carboxylic acid, cysteic acid, aldobionic acids, azelaic acid, azeloyl diglycinate, ascorbic acid, ascorbyl glucoside, sodium or magnesium ascorbyl phosphate, nicotinic acid and esters thereof, nicotinamide, nordihydroguaiaretic acid, urea, oligofucoses, cinnamic acid, hydroxystilbenes, resveratrol, glycosidases, stratum corneum chymotryptic enzyme, trypsin, chemotrypsin, sutilains, papain, bromelain, chelating agents, ethylenediaminetetraacetic acid, aminosulfonic chelating compounds, 4-(2-hydroxyethyl)piperazine-1-ethanesulfonic acid, sodium methylglycine diacetic acid, O-octanoyl-6-D-maltose, *N*-acetylglucosamine, Salicyloyl Phytosphingosine, papaya, pineapple, squash and sweet potato.

**17.** Dermopharmaceutical composition for use according to any one of claims 10 to 12, additionally comprising a dermopharmaceutically effective amount of at least one dermopharmaceutical adjuvant selected from the group consisting of calcium, retinol, trenitoin, calcitriol, calcipotriol, tacalcitol, beta-sitosterol sulfate and *Helix Aspersa Müller* glycoconjugates.

**18.** Dermopharmaceutical composition for use according to any one of claims 10 to 12, additionally comprising a dermopharmaceutically effective amount of at least one dermopharmaceutical adjuvant selected from the group consisting of allantoin, cadherins, integrins, selectins, hyaluronic acid receptors, immunoglobulins, fibroblast growth factor, connective tissue growth factor, platelet derived growth factor, vascular endothelial growth factor, epidermal growth factor, insulin-like growth factor, keratinocyte growth factor, colony stimulating factors, transforming growth factors-beta, tumor necrosis factor-alpha, interferons, interleukins, matrix metalloproteases, protein tyrosine phosphatase receptors, Tripeptide-10 Citrulline, *Centella asiatica, Rosa moschata, Echinaa angustifolia, Symphytum officinal, Equisetum arvense, Hypericum perforatum, Mimosa tenuiflora, Persea gratisima, Prunus africanum, Tormentilla erecta, Aloe vera,* and mixtures thereof.

Europäisches
Patentamt

European
Patent Office

Office européen
des brevets

**EUROPEAN SEARCH REPORT**

Application Number

EP 18 16 9832

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | T.E. GOTTSCHALCK, G.N. MCEWEN, JR.: "International Cosmetic Ingredient Dictionary and Handbook", 2006, THE COSMETIC, TOILETRY AND FRAGRANCE ASSOCIATION, WASHINGTON, D.C., XP002556695, * 11th edition ISBN 1-882621-36-0 Vol 2, p. 2015; column 2 * & T.E. GOTTSCHALCK, G.N. MCEWEN, JR.: "International Cosmetic Ingredient Dictionary and Handbook", 2006, THE COSMETIC, TOILETRY AND FRAGRANCE ASSOCIATION, WASHINGTON, D.C. * 11th edition ISBN 1-882621-36-0 Vol 3, pp. 2823-2825; page 2825, column 2 * | 1-18 | INV. A61K8/99 A61K8/73 A61Q19/00 A61P17/02 A61P17/06 A61K35/74 |
| X,D | EP 1 402 898 A (LIPOTEC SA [ES]) 31 March 2004 (2004-03-31) * the whole document * | 1-18 | TECHNICAL FIELDS SEARCHED (IPC) A61Q A61K |
| X | DE 10 2004 037753 A1 (HENKEL KGAA [DE]) 16 March 2006 (2006-03-16) * paragraphs [0118], [0119] * * paragraph [0094] * * paragraph [0009]; claim 1 * | 1-18 | |
| X | WO 2008/084890 A (STC NARA CO LTD [KR]; LEE KYE-HO [KR]; CHUNG SUNG-HO [KR]; SONG JI-HOO) 17 July 2008 (2008-07-17) * claims 1,3 * * page 1, lines 11-16 * * page 4, lines 16-22 * | 1-18 | |

-/--

The present search report has been drawn up for all claims

| Place of search | Date of completion of the search | Examiner |
|---|---|---|
| Munich | 30 May 2018 | Heller, Dorothée |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

2

Europäisches
Patentamt

European
Patent Office

Office européen
des brevets

**EUROPEAN SEARCH REPORT**

Application Number

EP 18 16 9832

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | EP 1 700 947 A (RESTANI MORENA [IT]; LENZI LUCIO [IT]) 13 September 2006 (2006-09-13) <br> * page 2, lines 48-52 * <br> * claims * <br> ----- | 1-18 | |
| A | MARIA NEVOT ET AL: "Ultrastructural Analysis of the Extracellular Matter Secreted by the Psychrotolerant Bacterium Pseudoalteromonas antarctica NF3", MICROBIAL ECOLOGY, SPRINGER-VERLAG, NE, vol. 51, no. 4, 28 April 2006 (2006-04-28), pages 501-507, XP019421791, ISSN: 1432-184X <br> * the whole document * <br> * page 501, paragraphs 2,3 * <br> ----- | 1-18 | |
| X,P | WO 2009/106343 A (LIPOTEC SA [ES]; CARRENO SERRAIMA CRISTINA [ES]; VAN DEN NEST WIM [ES]) 3 September 2009 (2009-09-03) <br> * claims 25,29 * <br> * examples 14,15 * <br> * page 33, lines 11,12,33-35 * <br> ----- | 1-18 | TECHNICAL FIELDS SEARCHED (IPC) |

The present search report has been drawn up for all claims

| Place of search | Date of completion of the search | Examiner |
|---|---|---|
| Munich | 30 May 2018 | Heller, Dorothée |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 18 16 9832

This annex lists the patent family members relating to the patent documents cited in the above-mentioned European search report.
The members are as contained in the European Patent Office EDP file on
The European Patent Office is in no way liable for these particulars which are merely given for the purpose of information.

30-05-2018

| Patent document cited in search report | | Publication date | Patent family member(s) | | Publication date |
|---|---|---|---|---|---|
| EP 1402898 | A | 31-03-2004 | AT | 285787 T | 15-01-2005 |
| | | | BR | 0210428 A | 17-08-2004 |
| | | | BR | PI0210428 B1 | 17-04-2018 |
| | | | CA | 2449910 A1 | 27-12-2002 |
| | | | DE | 60202474 D1 | 03-02-2005 |
| | | | DE | 60202474 T2 | 29-12-2005 |
| | | | EP | 1402898 A1 | 31-03-2004 |
| | | | ES | 2181592 A1 | 16-02-2003 |
| | | | ES | 2236525 T3 | 16-07-2005 |
| | | | JP | 4252444 B2 | 08-04-2009 |
| | | | JP | 2004534076 A | 11-11-2004 |
| | | | KR | 20040030675 A | 09-04-2004 |
| | | | MX | PA03011622 A | 01-07-2004 |
| | | | PT | 1402898 E | 31-05-2005 |
| | | | US | 2004242466 A1 | 02-12-2004 |
| | | | WO | 02102406 A1 | 27-12-2002 |
| DE 102004037753 A1 | | 16-03-2006 | DE | 102004037753 A1 | 16-03-2006 |
| | | | WO | 2006015675 A1 | 16-02-2006 |
| WO 2008084890 | A | 17-07-2008 | | | |
| EP 1700947 | A | 13-09-2006 | AT | 440994 T | 15-09-2009 |
| | | | EP | 1700947 A2 | 13-09-2006 |
| | | | ES | 2332374 T3 | 03-02-2010 |
| WO 2009106343 | A | 03-09-2009 | | | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- FR 2342059 A1
- WO 8704617 A1
- US 3920835 A
- US 3988470 A
- US 4234599 A
- US 4363815 A
- US 5886042 A
- US 6191167 A
- EP 0232982 A1
- EP 1032378 A1
- US 5407958 A1
- FR 2756489 A1
- US 20060269495 A1
- US 2006026949 A1
- FR 2761362 A1
- FR 2761363 A1
- FR 2767833 A1
- FR 2850024 A1
- FR 2888494 A1
- US 20030232042 A1
- US 20030232043 A1
- US 4224339 A
- US 6071962 A
- US 5847003 A
- US 4194007 A
- US 5728733 A
- US 5621008 A
- EP 0596838 A2
- EP 0628308 A1
- EP 0628309 A1
- EP 0631779 A1
- FR 2839449 A1
- US 5045559 A
- US 20030113352 A1
- US 20030229141 A1
- WO 9500136 A1
- US 5747482 A
- US 5948822 A
- US 20040138177 A1
- US 20080090772 A1
- FR 2816838 A1
- ES 2181592 A1
- EP 1700947 A2

**Non-patent literature cited in the description**

- **L. PONS ; J.L. PARRA.** Ciencia Cosmética: Bases fisiológicas y criterios prácticos" (Cosmetic Science: Physiological Bases and Practical Criteria). *Consejo General de Colegios Farmacéuticos,* 1995, 36-37
- The Biology of the Skin. Parthenon Publishing, 2001, 221
- Bioengineering of the Skin: Water and the Stratum Corneum. CRC Press, 1994, 3-12
- Ciencia Cosmética: Bases fisiológicas y criterios prácticos. **L. PONS ; J.L. PARRA.** Consejo General de Colegios Farmacéuticos. 1995, 71
- The Biology of the Skin. Parthenon Publishing, 2001, 225
- **L.G. KÖMÜVES.** *J. Invest. Dermatol.,* 2000, vol. 115, 361-67
- **H. MIYOSHI et al.** *J. Dermatol.,* 2005, vol. 32, 346-53
- **P.D. GUPTA et al.** *Current Science,* 2000, vol. 78, 1-5
- **J.N. KRAFT et al.** *Skin Therapy Letter,* 2005, vol. 10, 1
- **J.W. FLUHR et al.** *Acta Derm. Venerol.,* 1999, vol. 79, 418-21
- **M. GLOOR.** *Skin Pharmacol. Physiol.,* 2004, vol. 17, 267-73
- **K.C. MADISON.** *J. Invest. Dermatol.,* 2003, vol. 121, 231-41
- **G. SWANBECK.** *Acta Derm. Venerol.,* 1967, vol. 48, 123-7
- Tratado de Farmacia Galénica" (Treaty of Galenic Pharmacy). 1993
- **C.K. SCHAAB.** Impregnating Fabrics With Microcapsules. *HAPPI,* May 1986
- **G. NELSON.** *Int. J. Pharm.,* 2002, vol. 242, 55-62
- Biofunctional Textiles and the Skin. **U.C. HIPLER et al.** Curr. Probl. Dermatol. 2006
- **R.K. MALCOM et al.** *J. Cont. Release,* 2004, vol. 97, 313-320
- CTFA Cosmetic Ingredient Handbook. 2008